# EUROPEAN PATENT SPECIFICATION

(11) **EP 0 811 392 B1**
(45) Date of publication and mention of the grant of the patent: **31.10.2001**
(21) Application number: 96112401.3
(22) Date of filing: 01.08.1996
(51) Int. Cl.: A61L 15/46, A61L 15/20

(54) **Breathable absorbent article having a chelating agent based odour control system**
Atmungsfähiger absorbierender Artikel mit einem ein Chelatierungsmittel enthaltenden Geruchskontrollsystem
Article absorbant perméable à l'air contenant un système de contrôle des odeurs à base d'agent chélatant

(30) Priority: 07.06.1996 EP 96109172; 07.06.1996 EP 96109179; 07.06.1996 EP 96109178
(43) Date of publication of application: 10.12.1997
(73) Proprietor: THE PROCTER & GAMBLE COMPANY, Cincinnati, Ohio 45202 (US)
(72) Inventor: Guarracino, Mario, 64028 Silvi Marina (IT); Gagliardini, Alessandro, 60035 Jesi (IT); Carlucci, Giovanni, 66100 Chieti (IT); Cimini, Carmine, 65126 Pescara (IT)
(74) Representative: Hirsch, Uwe Thomas

(56) References cited:
- EP-A- 0 257 951
- US-A- 4 273 786
- US-A- 4 356 190

## Description

The present invention relates to absorbent articles, particularly sanitary napkins and panty liners which have an improved odour control system.

### Background of the Invention

Whilst the primary focus of absorbent articles, in particular sanitary napkins remains the ability of these articles to absorb and retain fluids, another important area of development in this field is the control of odourous compounds contained within the absorbed articles during their use. Malodourous compounds typically present in absorbent articles originate from a number of sources. Firstly, the actual components of the fluid discharge such as urine, perspiration, menstrual fluids, menstrual blood and vaginal discharges may themselves contain malodourous compounds. Secondly, malodourous compounds are also generated as a result of the degradation of the components of the fluid discharge contained within the absorbent articles. Consequently, there are a wide range of compounds which may be present at some time during the use of an absorbent article which have an associated malodour. These compounds include fatty acids, ammonia, amines, sulphur containing compounds, ketones and aldehydes and numerous derivatives thereof.

The presence and detection of malodourous compounds from absorbent articles during their use, particularly those associated with menstruation may cause the wearer of these products embarrassment. Thus, the prevention of the detection of malodour from such products is highly desirable.

As a result there are numerous disclosures in the art which describe various agents which provide odour control for use in absorbent articles. These odour control agents typically function by physical absorption of the odourous compound or by chemical interaction with the odourous or precursors of odourous compounds or by masking the odour.

The odour control agents are typically classified according to the type of odour the agent is intended to combat. Odours may be classified as being essentially acidic, basic or neutral. Acidic odour controlling agents have a pH greater than 7 and typically include inorganic carbonates, bicarbonates, phosphates and sulphates. Basic odour controlling agents have a pH of less than 7 and include compounds such as citric acid, boric acid and maleic acid. Neutral odour controlling agents have a pH of approximately 7 such as activated carbon, clays, zeolites, silicas and starches. Typically, the preferred agents utilised in absorbent articles are neutral odour controlling agents and mixtures thereof. Examples of such odour control agent are disclosed for example in EPO 348 978 and EPO 510 619.

Chelating agents are another example of odour control agents. These agents however function by chemical interaction with the precursors of malodorous compounds The use of chelants for the reduction of odours has been described in the art, for example US 4 356 190 discloses the use of aminopolycarboxylic compounds and aminophosphonates for inhibiting the production of undesirable products on body surfaces and their use in catamenial products. Also EPO 524 581 discloses the removal of odours by the formation of an insoluble complex with odour causing cations using compounds such as phosphates.

However, all of the above described odour control agents have associated drawbacks. Many odour control agents do not provide effective odour control over a range of odours. Alternatively, the more effective odour control agents are expensive or such as for example with activated carbon are aesthetically unappealing to the consumer. Also many odour controlling agents have problems related to their effective incorporation within the absorbent articles.

Hence, there still exists a need to provide alternative odour controlling agents or systems for effective utilization in absorbent articles. In particular, there exists a need to provide an odour control agent or system which addresses the problem of malodourous compound formation within absorbent articles by preventing the formation of the odour.

It has now been surprisingly found that the combination of a breathable absorbent article, particularly by the provision of a breathable backsheet together with an odour control system comprising a chelating agent provides an unexpected improvement in the odour control performance of the chelating agent odour control system.

The incorporation of breathable backsheets in absorbent articles for improved wearer comfort has been described in the art such as for example in GB 2 184 389, US 3 881 489 and EPO 203 821. US 4 059 114 discloses the incorporation of antimicrobial agents in sanitary napkins which have vapour permeable backsheets. However, none of these prior art documents recognise the benefits of the combination of a breathable absorbent article by the utilisation of a breathable backsheet, with a chelating agent odour control system.

It is believed that the synergic odour control performance benefit of a breathable absorbent article in combination with a chelating agent odour control agent is due to a number of factors.

Firstly, the breathability of the absorbent article results in increased movement of the volatile malodourous precursor compounds. Hence, the amount of actual physical contact between these compounds and the chelating agent odour control agents increases. Contact between the chelating agent odour control agents and the malodourous compounds is usually required in order to effectively combat the odourous compound. Frequently, large quantities of the odour control system is required within the absorbent article in order to ensure its effectiveness. This is because the odour control agents do not necessarily contact all the precursor malodourous compounds. The chelating agents however, whilst being a particularly effective odour controlling agent are expensive and thus it is desirable to avoid the necessity of such large quantities. In the present invention, the effectiveness of the odour control agent is significantly increased and thus the full capacity of the chelating agent odour control agent can be utilised. Hence less chelating agent may be required within the absorbent article whilst maintaining the required level of odour control.

Secondly, the breathability of the absorbent article reduces the hot humid and anaerobic environment between the skin of the wearer and the surface of the absorbent article. This hinders the growth of microorganisms, which are also known to be responsible for the generation of odourous compounds. Thus, the amount of odours associated with the presence of microorganisms is reduced by the absorbent articles of the present invention

Thirdly, the reduction in the hot, humid and occlusive environment between the vicinity of the skin of the wearer and the wearer facing surface of the absorbent article itself also reduces the tendency of the wearer of the product to perspire. Consequently, the amount of associated perspiration related odour will be reduced. Thus, the breathability of the article actually reduces the amount of odour generated within the absorbent article. As a result the odour control system works more effectively on the remaining odourous compounds present in the article.

In addition, due to the breathable nature of the absorbent article, the malodourous compounds contained therein may, similar to water vapour and air, be more readily exchanged with the environment. Hence, malodourous compounds are able to escape from the article and are dissipated into the surroundings. More importantly, the breathability of the article also allows the precursors compounds of malodourous compounds present in the article to escape from the absorbent article before degradation and hence before malodour formation takes place.

### Summary of the Invention

The present invention relates to an absorbent article, having a breathable backsheet and further comprising an odour control system comprising chelating agent. The combination of the chelating agent odour control system and the breathability of the absorbent article provides an unexpected improvement of the chelating agent odour control system performance.

### Detailed Description of the Invention

The present invention relates to breathable absorbent articles such as sanitary napkins, panty liners, incontinence devices and baby diapers. Typically such products comprise a liquid pervious topsheet, a backsheet and an absorbent core intermediate the topsheet and the backsheet. According to the present invention the breathability of the sanitary napkin is provided by the presence of a breathable backsheet which thereby allows the circulation of water vapour and preferably both water vapour and air through it. According to the present invention the absorbent article further comprises an odour control system comprising a chelating agent. It has now been found that a synergy exists between the breathability of the absorbent articles and the odour control system comprising chelating agent which results in an unexpected improvement of the performance of the chelating agent odour control system.

### Odour control system

According to the present invention the odour control system comprises as an essential component a chelating agent. Suitable chelating agents can be selected from the group consisting of amino carboxylates, amino phosphonates, polyfunctionally-substituted aromatic chelating agents and mixtures thereof, as hereinafter defined. Without intending to be bound by theory it is believed that the benefit of these materials is in part due to their exceptional ability to remove iron, copper, calcium, magnesium and manganese ions present in the absorbed fluids and their degradation products by the formation of chelates.

Amino carboxylates useful as chelating agents include ethylenediamine- tetracetates, N-hydroxyethylethylenediaminetriacetates, nitrilotriacetates, ethylene- diamine tetraproprionates, triethylenetetraaminehexacetates, diethy- lenetriamine pentaacetates, and ethanoldiglycines, alkali metal, ammonium, and substituted ammonium salts therein or mixtures therein.

Amino phosphonates are also suitable for use as chelating agents in the absorbent articles of the present invention and include ethylenediaminetetrakis (methylenephosphonates) as DEQUEST. Preferred, these amino phosphonates do not contain alkyl or alkenyl groups with more than about 6 carbon atoms.

Polyfunctionally-substituted aromatic chelating agents are also useful in the compositions herein for example see U.S. Patent 3,812,044, issued May 21, 1974, to Connor et al. Preferred compounds of this type, in acid form, are dihydroxydisulfobenzenes such as 1,2-dihydroxy-3,5-disulfobenzene.

A preferred biodegradable chelating agent for use herein is ethylenediamine disuccinate ("EDDS"), especially the [S,S] isomer as described in U.S. Patent 4,704,233, November 3, 1987, to Hartman and Perkins.

Preferably the chelating agent are selected from ethylenediaminetetracetate, -triacetate, -diacetate, and -monoacetate, N,N, disuccinic acid (sodium salt), ethylenediamine penta (methylene phosponic acid) (sodium salt) ethylenendiamine tetra (methylene phosphonic acid) or mixtures thereof. Most preferably the chelating agent is ethylenediamine tetracetate.

According to the present invention the absorbent articles typically comprise from 5gm⁻² to 300gm⁻², more preferably from 10gm⁻² to 180gm⁻², most preferably from 35m⁻² to 65gm⁻² basis weight of said chelating agent.

According to the present invention the odour control system may comprise in addition to said chelating agent additional odour control agents known in the art. Suitable agents include zeolites, activated carbon, clay, masking agents, absorbent gelling material, antimicrobials, silica, starch, cyclodextrin, buffer systems, ion exchange resins, carboxylic acids, carbonates, bicarbonates, phosphates, sulphates or mixtures thereof. Preferred additional odour controlling agents are zeolites, silica, activated carbon, AGM, or mixtures thereof. Preferred odour control systems for use herein include the following combinations such as chelating agent, zeolites and activated carbon; the combination of chelating agent, zeolites and silica and also the combination of chelating agent, zeolites, silica and absorbent gelling materials. An advantage of the combination of the chelating agent and additional odour control agents, in particular the absorbing materials such as AGM and silica in the breathable absorbent article is the promotion of improved dryness of the absorbent article.

According to the present invention the ratio of said chelating agent to said additional odour controlling agents is from 1:10 to 10:1, preferably from 1:5 to 5:1, more preferably from 1:3 to 3:1.

The chelating agent odour control system of the present invention may be incorporated into the absorbent article by any of the methods disclosed in the art, for example, the system may be layered on the core of the absorbent material or mixed within the fibres of the absorbent core. The odour control system is preferably incorporated between two layers of cellulose tissue. Optionally the odour control system may be bonded between two cellulose tissue layers with for example a hot melt adhesive or any suitable bonding system.

The chelating agent and additional optional odour control agents may be incorporated as a powder or a granulate within the absorbent article. When used in a granulate or particulate form the chelating agent and the optional odour control agents may be granulated separately and then mixed together or granulated together.

The chelating agent may be distributed homogeneously over the entire absorbent article or, in the secondary topsheet or, in at least one layer of the core or any mixtures thereof. If additional odour controlling agents are present, the chelating agent is positioned such that at least a portion of the fluid discharge comes into contact with the chelating agent before the odour control agent. More preferably, the chelating agent is located towards or within the topsheet itself and the additional odour control agents are located further away from the topsheet than the chelating agent, preferably towards the backsheet. In a preferred embodiment the chelating agent is located in a separate layer from the optional odour control agents. Most preferably the chelating agent is positioned within at least one of the topsheet layers and the odour control material is positioned within the core.

The chelating agent and optional odour control agents may be distributed homogeneously throughout the absorbent article, or within any one of the layers of the absorbent article. The odour control system may also be distributed substantially in the centre of the absorbent article or substantially on the edges of the absorbent article.

According to the present invention the amount of chelating agent odour control system incorporated into the absorbent article may be readily determined by the man skilled in the art and is to some extend dependent on the end use of the absorbent article and bearing in mind the absorbent article dimensions. Typically the absorbent article comprises from 5gm⁻² to 400gm⁻ ², more preferably from 100gm⁻² to 300gm⁻², most preferably from 150gm⁻² to 250gm⁻² basis weight of said odour control system. For example a sanitary napkin or panty liner may comprise from 0.25g to 5g, preferably from 0.4g to 3g, most preferably from 0.5g to 2.5g of said odour control system.

### Backsheet

According to the present invention, the absorbent articles comprise as an essential component a breathable backsheet. The primary role of the breathable backsheet is to prevent the extrudes absorbed and contained in the absorbent article from wetting articles that contact the absorbent article such as pyjamas and undergarments. In order to achieve this the backsheet typically extends across the whole of the absorbent structure and may extend into and form part of or all of sideflaps, side wrapping elements or wings. In addition to the prevention of liquid transport through the backsheet however, the breathable backsheet also permits the transfer of water vapour and preferably both water vapour and air through it and thus allows the circulation of air into and out of the backsheet and the absorbent article itself.

Suitable breathable backsheets for use herein include all breathable backsheets known in the art. In principle there are two types of breathable backsheets, single layer breathable backsheets which are breathable and impervious to liquids and backsheets having at least two layers, which in combination provide both breathability and liquid imperviousness.

Suitable single layer breathable backsheets for use herein include those described for example in GB A 2184 389, GB A 2184 390, GB A 2184 391, US 4 591 523, US 3 989 867 US 3 156 242 and European Patent Application number 95120653.1.

Suitable dual or multi layer breathable backsheets for use herein include those exemplified in US 3 881 489, US 4 341 216, US 4 713 068, US 4 818 600, EPO 203 821, EPO 710 471, EPO 710 472, European Patent Application numbers 95120647.3, 95120652.3, 95120653.1 and 96830097.0.

Particularly preferred are backsheets meeting the requirements as defined in European Patent Application number 96830343.8 and more preferably wherein the absorbent article also meets the requirements as described therein.

According to the present invention the breathable backsheet comprises at least one, preferably at least two water vapour permeable layers. Suitable water vapour permeable layers include 2 dimensional, planar micro and macro-porous films, monolithic films, macroscopically expanded films and formed apertured films. According to the present invention the apertures in said layer may be of any configuration, but are preferably spherical or oblong. The apertures may also be of varying dimensions. In a preferred embodiment the apertures are preferably evenly distributed across the entire surface of the layer, however layers having only certain regions of the surface having apertures is also envisioned.

2 dimensional planar films as used herein have apertures having an average diameter of from 5 micrometers to 200 micrometers. Typically, 2-dimensional planar micro porous films suitable for use herein have apertures having average diameters of from 150 micrometers to 5 micrometers, preferably from 120 micrometers to 10 micrometers, most preferably from 90 micrometers to 15 micrometers. Typical 2 dimensional planar macroporous films have apertures having average diameters of from 200 micrometers to 90 micrometers. Macroscopically expanded films and formed apertured films suitable for use herein typically have apertures having diameters from 100 micrometers to 500 micrometers. Embodiments according to the present invention wherein the backsheet comprises a macroscopically expanded film or an apertured formed film, the backsheet will typically have an open area of more than 5%, preferably from 10% to 35% of the total backsheet surface area.

Suitable 2 dimensional planar layers of the backsheet may be made of any material known in the art, but are preferably manufactured from commonly available polymeric materials. Suitable materials are for example GORE-TEX (TM) or Sympatex (TM) type materials well known in the art for their application in so-called breathable clothing. Other suitable materials include XMP-1001 of Minnesota Mining and Manufacturing Company, St. Paul, Minnesota, USA. As used herein the term 2 dimensional planar layer refers to layers having a depth of less than 1mm, preferably less than 0.5mm, wherein the apertures have an average uniform diameter along their length and which do not protrude out of the plane of the layer. The apertured materials for use as a backsheet in the present invention may be produced using any of the methods known in the art such as described in EPO 293 482 and the references therein. In addition, the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at their terminating ends. Preferably said protuberances are of a funnel shape, similar to those described in US 3, 929,135. The apertures located within the plane and the orifices located at the terminating end of protuberance themselves maybe circular or non circular, provided the cross sectional dimension or area of the orifice at the termination of the protuberance is smaller than the cross sectional dimension or area of the aperture located within the garment facing surface of the layer. Preferably said apertured preformed films are uni directional such that they have at least substantially, if not complete one directional fluid transport towards the core. Suitable macroscopically expanded films for use herein include films as described in for example in US 637 819 and US 4 591 523.

Suitable macroscopically expanded films for use herein include films as described in for example US 4 637 819 and US 4 591 523.

Suitable monolithic films include Hytrel™, available from DuPont Corporation, USA, and other such materials as described in Index 93 Congress, Session 7A "Adding value to Nonwovens", J-C. Cardinal and Y. Trouilhet, DuPont de Nemours International S.A., Switzerland.

According to the present invention the backsheet may comprise in addition to said water vapour permeable layer additional backsheet layers. Said additional layers may be located on either side of said water vapour permeable layer of the backsheet. The additional layers may be of any material, such as fibrous layers or additional water vapour permeable layers as described herein above.

According to the present invention the absorbent articles may further comprise a topsheet and absorbent core. The absorbent material or core can be a fluffy fibrous absorbent core, comprising hydrogel particles if desired, or laminated tissues with or without particulate materials including hydrogel particles. The absorbent core fibres can be any of those known in the art including cellulose fibres or polymeric fibres rendered absorbent or even non absorbent matrix fibres. Also tissues of sufficient basis weight and absorbency can be used in the absorbent core according to the present invention.

According to the present invention the topsheet may comprise a single layer or a multiplicity of layers. In a preferred embodiment the topsheet comprises a first layer which provides the user facing surface of the topsheet and a second layer between the first layer and the absorbent structure/core. The topsheet provides a layer through which the liquids to be absorbed penetrate to the absorbent material.

The topsheet as a whole and hence each layer individually needs to be compliant, soft feeling, and non-irritating to the wearer's skin. It also can have elastic characteristics allowing it to be stretched in one or two directions. Typically, the topsheet extends across the whole of the absorbent structure and can extend into and form part of or all of the preferred sideflaps, side wrapping elements or wings. According to the present invention the topsheet may be formed from any of the materials available for this purpose and known in the art, such as non woven fabrics, films or combinations of both. In a preferred embodiment of the present invention at least one of the layers of the topsheet comprises a hydrophobic, liquid permeable apertured polymeric film. Preferably, the upper layer is provided by a film material having apertures which are provided to facilitate liquid transport from the wearer facing surface towards the absorbent structure, as detailed for example in US 3 929 135, US 4 151 240, US 4 319 868, US 4 324 426, US 4 343 314 and US 4 591 523.

According to the present invention the absorbent article is constructed by joining the various elements such as topsheet, backsheet and absorbent core by any means well known in the art. For example the backsheet and/ or topsheet may be joined to the absorbent core or to each other by a uniform continuous layer of adhesive, a patterned layer of adhesive, or an array of separate lines, spirals or spots of adhesive. Alternatively, the elements may be joined by heat bonds, pressure bonds, ultra sonic bonds, dynamic mechanical bonds or any other suitable joining means known in the art and any combination thereof. Preferably the breathable backsheet is bonded to other elements of the absorbent article so as to minimise and preferably eliminate any reduction in the vapour permeability of the backsheet .

According to the present invention the absorbent article may find utility as sanitary napkins, panty liners, adult incontinence products and baby diapers. The present invention finds particular susceptibility as sanitary napkins and panty liners. Thus in addition to the components described herein above, the absorbent article may also comprise all those features and parts which are typical for products in the context of their intended use such as wings and side flaps, undergarment adhesive means, release paper, wrapping elements, fastening means and the like.

### Example 1:

This is an example of a panty liner according to the present invention and is a modified panty liner based on Always "Alldays Duo Active" manufactured by Procter & Gamble, Germany. The topsheet is a film/non woven composite {film supplier code BPC 5105 CPM BP Chemical Germany, non woven supplier code ARBO TB/BI Mequinenza Spain}. The core material is a tissue laminate (13.2 cm x 4.0 cm) composed of a 2 layers of airlayed tissue of 55 g/m² basis weight {available from Unikay Italy under the supplier code Unikay 303 LF}. Between the two tissue layers the laminate contains an odour control system of AGM (available from DOW Chemicals Germany under the supplier code; DOW XZ 95890.1) at a basis weight of 67 g/m², zeolite (available from Degussa Germany under the supplier code; Wessalith CS) at a basis weight of 50 g/m² and chelating agent {ethylenediamine tetraacetate available from BASF AG, under the code name TRILON B polvere} at a basis weight of 50 g/m².

The backsheet comprises two layers a first layer and a second layer. The first layer is in contact with the absorbent tissue and the second layer. The second layer is in contact with the first layer and the undergarment of the wearer. The first layer is a formed apertured film (CPT) made of Low Density PE {supplied by Tredegar Film Products B.V. Holland under the manufacturing code X-1522}. The second layer is composed of a nonwoven laminate {14MB/14SB manufactured by Corovin GmbH in Germany under the trade name MD 2005}. The nonwoven laminate is composed of 14 g/m² spunbond and 14 g/m² meltblown. Each backsheet layer is joined over the full surface by a extensively overlapped spiral glue application at a basis weight of approximately 8 g/m². The glue utilised for attachment of both backsheet layers was supplied by SAVARE' SpA. Italy (under the material code PM17).

### Example 2:

Example 2 is identical to example 1 except that the second layer of the backsheet has been replaced by a nonwoven laminate composed of 16g/m² spunbond and 6 g/m² meltblown {supplied under the code of SM 22-6PH by Union SpA, Italy}.

### Example 3:

This is an example of a sanitary napkin according to the present invention. The sanitary napkin is based on an Always Ultra sanitary napkin available from Procter & Gamble Germany which has been modified. The topsheet is a CPM material available from Tredegar Film Products B. V. Holland under the code X-1522. The core material is a tissue laminate (20.7 cm x 7.0 cm) composed of a 2 layers of airlayed tissue of 55 g/m² basis weight {available from Unikay Italy under the supplier code Unikay 303 LF}. Between the two tissue layers the laminate contains an odour control system of AGM (available from DOW Chemicals Germany under the supplier code; DOW XZ 95890.1) at a basis weight of 64 g/m², a zeolite (available from Degussa Germany under the supplier code; Wessalith CS) at a basis weight of 61 g/m² and chelating agent (ethylenediamine tetraacetate available from BASF AG, Germany under the supplier code; TRILON B polvere) at a basis weight of 65g/m². The core laminate was manufactured and supplied by Korma Italy (under the experimental manufacturing code: XA 070.01.003). The sanitary napkin has a multi-layer breathable backsheet comprising a formed apertured film backsheet layer and a second nonwoven layer. The first layer is a blend of low and high density PE with a crush resistant hexagonal hole configuration {supplied by Tredegar Film Products B.V. Holland under the manufacturing code AS 225 HD 25}. The second layer is an improved nonwoven laminate composed of 3 layers with basis weights 14g/m² spunbond - 20 g/m² meltblown - 14 g/m² spunbond (manufactured by Corovin GmbH in Germany under the trade name MD 3005).

## Claims

1. An absorbent article comprising a liquid permeable topsheet, a breathable backsheet and an absorbent core, said core being intermediate said topsheet and said core, said absorbent article comprising an odour control system comprising chelating agent .

2. An absorbent article according to claim 1, wherein said chelating agent is selected from aminocaboxylates, aminophosphates, polyfunctionally substituted aromatics or mixtures thereof.

3. An absorbent article according to any one of the preceding claims, wherein said chelating agent is selected from ethylene diamine tetracetate, or N,N, disuccinic acid, or ethylenediamine penta (methylene phosphonic acid), or ethylenediamine tetra (methylene phosphonic acid) or mixtures thereof.

4. An absorbent article according to any one of the preceding claims, wherein said chelating agent is ethylenediamine tetracetate.

5. An absorbent article according to any one of the preceding claims, wherein said odour control system further comprises at least one odour control agent selected from zeolite, absorbent gelling materials, silica, activated carbon and mixtures thereof.

6. An absorbent article according to claim 5, wherein said odour control system further comprises zeolite, silica and absorbent gelling materials.

7. An absorbent article according to claim 5, wherein said odour control system further comprises zeolite and activated carbon.

8. An absorbent article according to any one of the preceding claims, wherein said absorbent article comprises from 5gm⁻² to 300gm⁻² of said chelating agent.

9. An absorbent article according to any one of the preceding claims, wherein said breathable backsheet comprises at least one layer selected from an apertured polymeric film or a 2-dimensional planar apertured film.

10. An absorbent article according to claim 9, wherein said layer is a 2 dimensional planar apertured layer, wherein said apertures have an average diameter of from 150 micrometers to 5 micrometers.

11. An absorbent article according to claim 9, wherein said layer is an apertured polymeric film, wherein said apertures have an average diameter of from 100 micrometers to 500 micrometers.

12. An absorbent article according to claim 9, wherein said breathable backsheet comprises at least two layers, a first layer comprising an apertured layer and a second layer comprising a fibrous layer.

13. An absorbent article according to any one of the preceding claims, wherein said article is a sanitary napkin or a panty liner.

14. An absorbent article according to claim 13, wherein said absorbent article comprises from 0.25g to 5g by weight of said odour control system

## Patentansprüche

1. Absorbierender Artikel, der eine flüssigkeitsdurchlässige Oberschicht, eine luftdurchlässige Unterschicht und einen absorbierenden Kern umfaßt, wobei der Kern zwischen der Oberschicht und der Unterschicht liegt, wobei der absorbierende Artikel ein Geruchsbekämpfungssystem, das einen Chelatbildner aufweist, umfaßt.

2. Absorbierender Artikel nach Anspruch 1, wobei der Chelatbildner aus Aminocaboxylaten, Aminophosphaten, polyfunktionell substituierten Aromaten oder Mischungen daraus ausgewählt ist.

3. Absorbierender Artikel nach einen der vorhergehenden Ansprüche, wobei der Chelatbildner aus Ethylen-Diamin-Tetracetat oder N, N, Di-Bernsteinsäure oder Ethylendiaminpenta-(Methylen-Phosphonsäure) oder Ethylen-Diamin-Tetra-(Methylen-Phosphonsäure) oder Mischungen daraus ausgewählt wird.

4. Absorbierender Artikel nach einem der vorhergehenden Ansprüche, wobei der Chelatbildner Ethylendiamintetracetat ist.

5. Absorbierender Artikel nach einem der vorhergehenden Ansprüche, wobei das Geruchsbekämpfungssystem weiter mindestens ein Geruchsbekämpfungsmittel, das aus Zeolit, absorbierenden Geliermaterialien, Silika, Aktivkohle und Mischungen daraus besteht, umfaßt.

6. Absorbierender Artikel nach Anspruch 5, wobei das Geruchsbekämpfungssystem weiter Zeolit, Silika und absorbierende Geliermaterialien umfaßt.

7. Absorbierender Artikel nach Anspruch 5, wobei das Geruchsbekämpfungssystem weiter Zeolit und Aktivkohle umfaßt.

8. Absorbierender Artikel nach einem der vorhergehenden Ansprüche, wobei der absorbierende Artikel 5 gm⁻² bis 300 gm⁻² des Chelatbildners umfaßt.

9. Absorbierender Artikel nach einem der vorhergehenden Ansprüche, wobei die luftdurchlässige Unterschicht mindestens eine Schicht umfaßt, die aus einem mit Öffnungen versehenen Polymerfilm oder einem zweidimensionalen planaren mit Öffnungen versehenen Film besteht.

10. Absorbierender Artikel nach Anspruch 9, wobei die Schicht eine zweidimensionale planare mit Öffnungen versehene Schicht ist, wobei die Öffnungen einen mittleren Durchmesser von 150 Mikrometer bis 5 Mikrometer aufweisen.

11. Absorbierender Artikel nach Anspruch 9, wobei die Schicht ein mit Öffnungen versehener Polymerfilm ist, wobei die Öffnungen einen mittleren Durchmesser von 100 Mikrometern bis 500 Mikrometern aufweisen.

12. Absorbierender Artikel nach Anspruch 9, wobei die luftdurchlässige Unterschicht mindestens zwei Schichten umfaßt, eine erste Schicht, die eine mit Öffnungen versehene Schicht umfaßt, und eine zweite Schicht, die eine Faserschicht umfaßt.

13. Absorbierender Artikel nach einem der vorhergehenden Ansprüche, wobei der Artikel eine Damenbinde oder eine Slipeinlage ist.

14. Absorbierender Artikel nach Anspruch 13, wobei der absorbierende Artikel 0,25 g bis 5 g des Geruchsbekämpfungssystems umfaßt.

## Revendications

1. Article absorbant comprenant une feuille de dessus perméable aux liquides, une feuille de fond pouvant respirer et une âme absorbante, ladite âme étant placée entre ladite feuille de dessus et ladite feuille de fond, ledit article absorbant comprenant un système de limitation d'odeur comprenant un agent chélatant.

2. Article absorbant selon la revendication 1, dans lequel ledit agent chélatant est choisi parmi les aminocarboxylates, les aminophosphates, les composés aromatiques substitués de manière polyfonctionnelle, ou leurs mélanges.

3. Article absorbant selon l'une quelconque des revendications précédentes, ' dans lequel ledit agent chélatant est choisi parmi l'éthylènediamine tétraacétate, ou l'acide N,N-disuccinique, ou l'acide éthylènediamine penta(méthylène phosphonique) ou l'acide éthylènediamine tétra(méthylène phosphonique), ou leurs mélanges.

4. Article absorbant selon l'une quelconque des revendications précédentes, dans lequel ledit agent chélatant est l'éthylènediamine tétraacétate.

5. Article absorbant selon l'une quelconque des revendications précédentes, dans lequel ledit système de limitation d'odeur comprend, en outre, au moins un agent de limitation d'odeur choisi parmi la zéolite, les matériaux absorbants gélifiants, la silice, le charbon activé, et leurs mélanges.

6. Article absorbant selon la revendication 5, dans lequel ledit système de limitation d'odeur comprend, en outre, de la zéolite, de la silice et les matériaux absorbants gélifiants.

7. Article absorbant selon la revendication 5, dans lequel ledit système de limitation d'odeur comprend, en outre, de la zéolite et le charbon activé.

8. Article absorbant selon l'une quelconque des revendications précédentes, dans lequel ledit article absorbant comprend de 5 gm⁻² à 300 gm⁻² dudit agent chélatant.

9. Article absorbant selon l'une quelconque des revendications précédentes, dans lequel ladite feuille de fond pouvant respirer comprend au moins une couche choisie entre un film polymère perforé ou un film plan à deux dimensions perforé.

10. Article absorbant selon la revendication 9, dans lequel ladite couche est une couche plane à deux dimensions perforée, dans lequel lesdits orifices ont un diamètre moyen compris entre 150 micromètres et 5 micromètres.

11. Article absorbant selon la revendication 9, dans lequel ladite couche est un film polymère perforé, dans lequel lesdits orifices ont un diamètre moyen compris entre 100 micromètres et 500 micromètres.

12. Article absorbant selon la revendication 9, dans lequel ladite feuille de fond pouvant respirer comprend au moins deux couches, une première couche comprenant une couche perforée et une deuxième couche comprenant une couche fibreuse.

13. Article absorbant selon l'une quelconque des revendications précédentes, dans lequel ledit article est une serviette hygiénique ou une garniture de culotte.

14. Article absorbant selon la revendication 13, dans lequel ledit article absorbant comprend de 0,25 g à 5 g en poids dudit système de limitation d'odeur.
